# EUROPÄISCHE PATENTSCHRIFT

(11) **EP 0 797 429 B1**
(45) Veröffentlichungstag und Bekanntmachung des Hinweises auf die Patenterteilung: **07.11.2001**
(21) Anmeldenummer: 96933343.4
(22) Anmeldetag: 20.09.1996
(51) Int. Cl.: A61K 9/00

(54) **OSMOTISCHE VORRICHTUNG ZUR KONTINUIERLICHEN FREISETZUNG VON WIRKSTOFFEN IN DIE FLÜSSIGKEITEN DES GASTROINTESTINALTRAKTES**
OSMOTIC DEVICE FOR THE CONTINUOUS RELEASE OF ACTIVE SUBSTANCES INTO THE FLUIDS OF THE GASTRO-INTESTINAL TRACT
DISPOSITIF OSMOTIQUE POUR LA LIBERATION EN CONTINU DE SUBSTANCES ACTIVES DANS LES LIQUIDES DU TRACTUS GASTRO-INTESTINAL

(30) Priorität: 05.10.1995 DE 19537090
(43) Veröffentlichungstag der Anmeldung: 01.10.1997
(73) Patentinhaber: LTS LOHMANN Therapie-Systeme AG, 56626 Andernach (DE)
(72) Erfinder: CREMER, Karsten, 53119 Bonn (DE)
(74) Vertreter: Flaccus, Rolf-Dieter, Dr.
(86) Internationale Anmeldenummer: EP9604117
(87) Internationale Veröffentlichungsnummer: WO9712599

(56) Entgegenhaltungen:
- EP-A- 0 037 622
- FR-A- 2 319 380
- GB-A- 2 182 559
- US-A- 3 924 622

## Beschreibung

Die Erfindung betrifft eine osmotische Vorrichtung zur kontrollierten Freisetzung von Wirkstoffen in die Flüssigkeiten des Gastrointestinaltraktes durch eine in der äußeren Membran der Vorrichtung angeordnete Austrittsöffnung.

Solche osmotischen Vorrichtungen stellen eine Kategorie der sogenannten therapeutischen Systeme zur peroralen Anwendung dar (K.H. Bauer et al,. Pharmazeutische Technologie, S. 554-561, Thieme Verlag, 1986). Sie sind in der Lage, wesentlich kontrollierter als beispielsweise konventionelle Retard-Arzneiformen Wirkstoffe abzugeben.

Die Funktionsweise einer derartigen osmotischen Vorrichtung läßt sich am Beispiel des einfachsten möglichen Aufbaus, des Einkammer-Systems, verdeutlichen. Ein solches besteht aus einem den Wirkstoff enthaltenden, osmotisch aktiven Kern, der mit einer semipermeablen Polymermembran umhüllt ist. Die Polymermembran weist eine kleine Öffnung auf, durch die der Wirkstoff an die Umgebungsflüssigkeit abgegeben wird. Der Kern der Vorrichtung wird in der Regel aus einer Mischung aus Wirkstoff und Hilfsstoffen als Tablette gepresst. Die Polymermembran wird beispielsweise durch ein Sprühverfahren hergestellt, mit dem das Polymer mit weiteren Hilfsstoffen als Lösung oder Dispersion auf den Kern aufgebracht und anschließend getrocknet wird. Die Austrittsöffnung in der Polymermembran wird durch einen Laserstrahl erzeugt.

Nach der peroralen Applikation eines osmotischen Therapie-Systems in den Intestinaltrakt diffundiert aus diesem Flüssigkeit durch die Polymermembran in den wirkstoffhaltigen Kern. Der Wirkstoff und/oder ein dafür vorgesehener, osmotisch aktiver Hilfsstoff beginnen sich darin zu lösen. Hierdurch entsteht im Innenraum der Vorrichtung ein gegenüber dem Außenmedium erhöhter osmotischer Druck, der dazu führt, daß wirkstoffhaltige Lösung durch die Austrittsöffnung nach außen gedrückt wird. Weil sich das Nachdiffundieren von Flüssigkeit durch die Polymermembran in den Kern kontinuierlich fortsetzt, erfolgt der Druckausgleich durch Austritt von Wirkstofflösung mit großer Gleichmäßigkeit, solange Wirkstoff im Kern vorhanden ist. Die Geschwindigkeit der Wirkstoffabgabe läßt sich über die Zusammensetzung, Beschaffenheit und Ausdehnung der Membran sowie über die Löslichkeit der Bestandteile des Kerns mit Vorteil einstellen.

Perorale osmotische Therapie-Systeme können jedoch neben dem Vorteil einer Kontrolle der Freisetzungsgeschwindigkeit den Nachteil eines Schädigungspotentials für die Darmschleimhaut aufweisen. Dabei können die Schädigungspotentiale der oft in hochkonzentrierter Form freigesetzten Wirkstoffe und Hilfsstoffe infolge der Fokussierung durch die kleine Austrittsöffnung erheblich vergrößert werden, sodaß die Darmwand punktuell gravierend beschädigt wird.

Eine erste in Deutschland therapeutisch eingesetzte osmotische Vorrichtung mußte aufgrund der durch sie hervorgerufenen schweren Darmwandschädigungen im Jahre 1983 wieder vom Markt genommen werden. Das Schädigungspotential sowohl des in dem Produkt enthaltenen Wirkstoffes Indometacin wie auch des osmotisch aktiven Hilfsstoffes Kaliumchlorid war besonders groß.

Es ist jedoch auch beim Einsatz weniger schleimhautschädigender osmotischer Therapie-Systeme nicht auszuschließen, daß durch einen Fokussierungseffekt der Austrittsöffnung insbesondere bei vorgeschädigten Patienten Läsionen hervorgerufen werden.

Darüber hinaus wäre es wünschenswert, auch solche Wirksubstanzen in osmotischen Systemen verabreichen zu können, welche zwar ein vergleichsweise geringes schleimhautschädigendes Potential besitzen, jedoch nur in einem solchen Ausmaß, welches eine orale Verabreichung nach Abwägung von Nutzen und Risiken noch sinnvoll erscheinen läßt. Diese Substanzen sind nach dem heutigen Stand der Technik in konventionellen oralen Darreichungsformen verabreichbar, nicht aber in peroralen osmotischen Therapie-Systemen.

Der Erfindung liegt die Aufgabe zugrunde, eine osmotische Vorrichtung zur kontrollierten Freisetzung von Wirkstoffen in die Flüssigkeiten des Gastrointestinaltraktes durch eine in der äußeren Membran der Vorrichtung angeordnete Austrittsöffnung der im Oberbegriff von Anspruch 1 genannten Art anzugeben und diese gegenüber dem Stand der Technik soweit zu verbessern, daß die Gefahr einer Schädigung der Darmschleimhaut möglichst weitgehend vermieden und auch bei vorgeschädigten Patienten keine Läsionen hervorgerufen werden.

Die Lösung der Aufgabe gelingt durch eine erfindungsgemäße Positionierung der Austrittsöffnung für Wirkstoff in Kombination mit einer speziellen Formgebung der osmotischen Vorrichtung nach Anspruch 1.

Mit Vorteil ist dabei die Vorrichtung derart gestaltet, daß die Austrittsöffnung neben abstandshaltend gegenüber der Schleimhautoberfläche ausgebildeten Bereichen der Vorrichtung an einer relativ dazu zurückliegenden Stelle der äußeren Membran im Abstand zur Oberfläche der Schleimhaut angeordnet ist.

Dabei kann die Vorrichtung Vertiefungen, konkave Krümmungen, gekrümmte oder gewinkelte Achsen, Ringförmigkeit oder sonstige Gestaltungsmerkmale aufweisen, bei welchen direkter oder enger Kontakt der Austrittsöffnung mit der Oberfläche der den Gastrointestinaltrakt auskleidenden Schleimhaut nicht möglich ist. Die Erfindung sieht somit vor, daß eine Austrittsöffnung für Wirkstofflösung zwangsweise im Abstand von Oberflächenregionen der Darmwand positioniert ist, indem zwischen der Austrittsöffnung und dem Teil des angrenzenden Intestinaltraktes ein Abstand eingehalten wird. Dieser Abstand führt dazu, daß die Wirkstofflösung nicht in derselben hohen Konzentration, in welcher sie die Austrittsöffnung passiert, auf eine kleine, der Austrittsöffnung entsprechende Fläche der Darmschleimhaut trifft, sondern auf eine größere Fläche derselben, sowie in verdünnter Form. Wirk- oder Hilfsstoffe mit schleimhautschädigendem Potential erreichen nach Applikation einer erfindungsgemäßen Vorrichtung die gastrointestinale Schleimhaut nur nach vorheriger Verdünnung und können - wenn überhaupt - diese nur erheblich weniger schädigen als herkömmliche osmotische Therapie-Systeme. Durch eine erfindungsgemäße Vorrichtung bleiben daher die o.g. Vorteile der osmotischen Therapie-Systeme erhalten, während die Arzneimittelsicherheit bei ihrer Anwendung erheblich verbessert wird.

Weitere Einzelheiten, Merkmale und Vorteile der Erfindung ergeben sich aus der nachstehenden Erläuterung einiger in den Zeichnungen schematisch dargestellter Ausführungsbeispiele.

Es zeigen:
- Fig. 1:: In perspektivischer Darstellung eine Vorrichtung in Form eines Ringes mit kreisförmigem oder annähernd kreisförmigen Querschnitt;
- Fig. 2:: Im Schnitt eine innerhalb der Darmwand lagernde Vorrichtung;
- Fig. 3:: Im Schnitt eine andere Ausführungsform der Vorrichtung;
- Fig. 4:: In perspektivischer Ansicht eine weitere Ausführung der Vorrichtung.

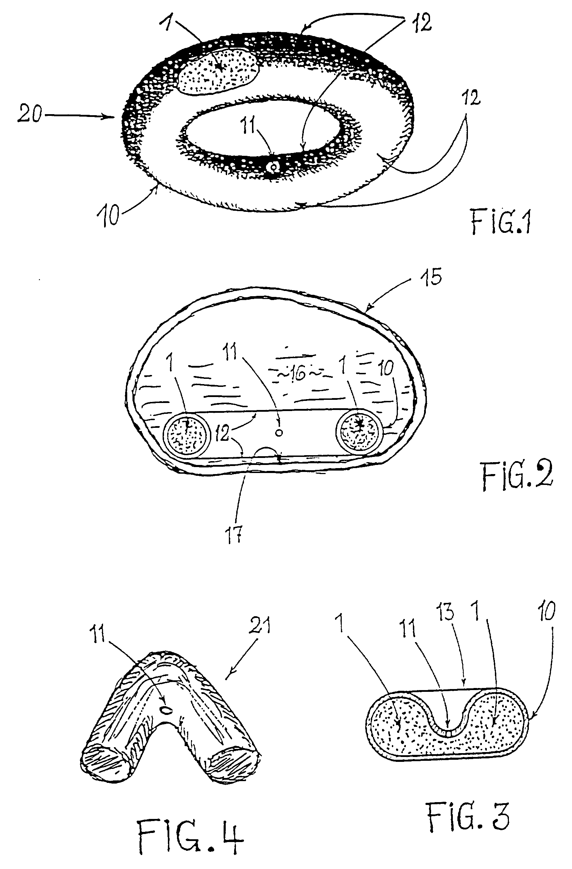

Die Zusammenschau der Fig. 1 bis 4 zeigt osmotische Vorrichtungen zur Therapierung der Schleimhaut (17) des Gastrointestinaltraktes mittels osmotisch gesteuerter, kontrollierter Freisetzung von Wirkstoff in der Flüssigkeit (16) des Gastrointestinaltraktes durch eine in der äußeren Membran (10) der Vorrichtung angeordnete Austrittsöffnung (11).

Die Vorrichtung besteht aus einem den Wirkstoff enthaltenden osmotischen Kern (1), der mit einer semipermeablen Polymermembran (10) umhüllt ist. Die Polymermembran (10) weist eine Austrittsöffnung (11) mit vergleichsweise kleinem Durchmesser auf, durch welche in Flüssigkeit des Gastrointestinaltraktes gelöster Wirkstoff an die Umgebungsflüssigkeit (16) abgegeben wird. Der Kern (1) der Vorrichtung wird in der Regel aus einer Mischung von Wirkstoff und Hilfsstoffen als Tablette gepresst. Die Polymermembran (10) kann beispielsweise durch ein Sprühverfahren hergestellt werden, mit welchem das Polymer gegebenenfalls mit weiteren Hilfsstoffen als Lösung oder Dispersion auf den Kern (1) aufgebracht und anschließend getrocknet wird. Die Austrittsöffnung (11) in der Polymermembran (10) wird durch einen Laserstrahl erzeugt.

In der Fig. 1 ist ein Ausführungsbeispiel der osmotischen Vorrichtung gezeigt, die in Form eines Ringes (20) mit kreisförmigem oder annähernd kreisförmigem Querschnitt ausgebildet ist, wobei die Austrittsöffnung (11) beispielsweise bei Anlage der Vorrichtung an der Oberfläche (17) der Schleimhaut einer Darmwand (15) (Fig. 2) im Abstand zur Oberfläche (17) der Schleimhaut am inneren Wandbereich des Ringes (20) angeordnet ist. Figur 1 zeigt weiter den bekannten Aufbau aus einem Wirkstoff und Hilfsstoff enthaltenden Kern (1) in einer Umhüllung durch die Membran (10), wobei die Austrittsöffnung (11) neben abstandshaltend gegenüber der Oberfläche (17) der Schleimhaut ausgebildeten Bereichen (12) der Vorrichtung an einer relativ dazu zurückliegenden Stelle der äußeren Membran (10) im Abstand zur Oberfläche der Schleimhaut angeordnet ist.

In der Fig. 2 ist die Position der Vorrichtung innerhalb eines im Schnitt dargestellten Darmabschnitts mit der Darmwand (15) dargestellt. Die aus Kern (1) und umhüllender Polymermembran (10) bestehende Vorrichtung weist die Austrittsöffnung (11) neben abstandshaltend gegenüber der Oberfläche (17) der Schleimhaut ausgebildeten Bereichen (12) an einer relativ dazu zurückliegenden Stelle der äußeren Membran (10) im Abstand zur Oberfläche (17) der Schleimhaut auf.

Mit (16) ist die Darmflüssigkeit innerhalb des Gastrointestinaltraktes angedeutet. Diese kann durch die Polymermembran (10) hindurchdiffundieren und löst dann Anteile des Wirkstoffs bzw. der hinzugefügten Hilfsstoffe in einem kontinuierlichen Vorgang auf, wobei im Innern der Vorrichtung ein osmotischer Druck entsteht, der die wirkstoff- und hilfsstoffhaltige Lösung durch die Austrittsöffnung (11) in an sich bekannter Weise austreten läßt.

Die Lehre der Erfindung, daß die Austrittsöffnung (11) infolge der besonderen Gestaltung der Vorrichtung bei wie auch immer gearteter Anlage an der Oberfläche (17) der Schleimhaut der Darmwand (15) im Abstand zur Oberfläche (17) der Schleimhaut angeordnet ist, kann verständlicherweise durch eine Vielzahl von entsprechend ausgebildeten äußeren Formen der Vorrichtung verwirklicht werden.

Außer der Form eines Ringes kommt die Form einer Ellipse, eines Ellipsoids oder von Abschnitten eines Ringes oder einer Ellipse bzw. eines Ellipsoids ebenso in Frage wie polygonale Formen und dergleichen.

Beispielsweise kann die Austrittsöffnung (11), wie dies die Fig. 3 zeigt, am Grunde einer Vertiefung zwischen abstandshaltenden Erhöhungen (13) angeordnet sein. Die Austrittsöffnung (11) kann aber auch in einem konkaven Bereich der Vorrichtung angeordnet sein. Weiterhin kann beispielsweise die Vorrichtung gemäß Fig. 4 die Form einer Keule (21) mit gekrümmter oder gewinkelter Achse aufweisen, wobei dann die Austrittsöffnung (11) im inneren Bereich der Krümmung oder des Winkels angeordnet ist.
Die Vorrichtung ist unkompliziert, mit wirtschaftlichen Mitteln herstellbar und erfüllt in optimaler Weise die eingangs genannten Aufgabe.

## Patentansprüche

1. Osmotische Vorrichtung zur kontrollierten Freisetzung von Wirkstoffen in die Flüssigkeit (16) des Gastrointestinaitraktes durch eine in der äußeren Membran (10) der Vorrichtung angeordnete Austrittsöffnung (11), **dadurch gekennzeichnet, daß** die Austrittsöffnung (11) neben abstandshaltend gegenüber der Oberfläche (17) der Schleimhaut ausgebildeten Bereichen (12,13) der Vorrichtung an einer relativ dazu zurückliegenden Stelle der äußeren Membran (10) im Abstand zur Oberfläche (17) der Schleimhaut angeordnet ist.

2. Vorrichtung nach Anspruch 1, **dadurch gekennzeichnet, daß** sie in Form eines Ringes (20) mit kreisförmigem oder annähernd kreisförmigem Querschnitt ausgebildet und die Austrittsöffnung (11) am inneren Wandbereich des Ringes (20) angeordnet ist.

3. Vorrichtung nach Anspruch 1 oder 2, **dadurch gekennzeichnet, daß** sie in Form eines Ringsegmentes ausgebildet ist.

4. Vorrichtung nach Anspruch 1, **dadurch gekennzeichnet, daß** sie in Form einer Elipse oder eines Elipsoids mit kreisförmigem oder annähernd kreisförmigem Querschnitt ausgebildet und die Austrittsöffnung (11) am inneren Wandbereich der Ellipse bzw. des Ellipsoids angeordnet ist.

5. Vorrichtung nach Anspruch 4, **dadurch gekennzeichnet, daß** sie in Form eines Segments der Ellipse bzw. des Ellipsoids ausgebildet ist.

6. Vorrichtung nach Anspruch 1, **dadurch gekennzeichnet, daß** die Austrittsöffnung (11) am Grunde einer Vertiefung angeordnet ist.

7. Vorrichtung nach Anspruch 1, **dadurch gekennzeichnet, daß** die Austrittsöffnung (11) in einem konkaven Bereich derselben angeordnet ist.

8. Vorrichtung nach Anspruch 1, **dadurch gekennzeichnet, daß** diese die Form einer Keule (21) mit gekrümmter oder gewinkelter Achse aufweist und die Austrittsöffnung (11) im inneren Bereich der Krümmung oder des Winkels angeordnet ist.

## Claims

1. Osmotic device for the controlled release of active compounds into the fluid (16) of the gastrointestinal tract through an outlet opening (11) arranged in the outer membrane (10) of the device, **characterized in that** the outlet opening (11), compared with areas (12, 13) of the device maintaining a distance relative to the surface (17) of the mucous membrane, is arranged in a position of the external membrane (10) at a distance from the surface (17) of the mucous membrane which, relatively thereto, is further away.

2. Device according to Claim 1, **characterized in that** it is designed in the form of a ring (20) having a circular or approximately circular cross-section and the outlet opening (11) is arranged on the inner wall area of the ring (20).

3. Device according to Claim 1 or 2, **characterized in that** it is designed in the form of a ring segment.

4. Device according to Claim 1, **characterized in that** it is designed in the form of an ellipse or of an ellipsoid with a circular or approximately circular cross-section and the outlet opening (11) is arranged on the inner wall area of the ellipse or of the ellipsoid.

5. Device according to Claim 4, **characterized in that** it is designed in the form of a segment of the ellipse or of the ellipsoid.

6. Device according to Claim 1, **characterized in that** the outlet opening (11) is arranged at the bottom of a hollow.

7. Device according to Claim 1, **characterized in that** the outlet opening (11) is arranged in a concave area thereof.

8. Device according to Claim 1, **characterized in that** this has the form of a body (21) having a curved or angled axis and the outlet opening (11) is arranged in the inner area of the curve or of the angle.

## Revendications

1. Dispositif osmotique pour la libération contrôlée de substances actives dans le liquide (16) du tractus gastro-intestinal à travers une ouverture de sortie (11) disposée dans la membrane extérieure (10) du dispositif, **caractérisé en ce que** l'ouverture de sortie (11) est disposée à distance de la surface (17) de la membrane, à côté de régions (12, 13) du dispositif configurées comme écarteurs par rapport à la surface (17) de la muqueuse, en un emplacement de la membrane extérieure (10) situé en retrait par rapport à la muqueuse.

2. Dispositif selon la revendication 1, **caractérisé en ce qu'**il est configuré en forme d'anneau (20) de section transversale circulaire ou sensiblement circulaire, l'ouverture de sortie (11) étant disposée dans la zone intérieure de la paroi de l'anneau (20).

3. Dispositif selon la revendication 1 ou 2, **caractérisé en ce qu'**il est configuré en forme de segment d'anneau.

4. Dispositif selon la revendication 1, **caractérisé en ce qu'**il est configuré en forme d'ellipse ou d'ellipsoïde de section transversale circulaire ou sensiblement circulaire, l'ouverture de sortie (11) étant disposée sur une zone intérieure de la paroi de l'ellipse ou de l'ellipsoïde.

5. Dispositif selon la revendication 4, **caractérisé en ce qu'**il est configuré en forme de segment d'ellipse ou d'ellipsoïde.

6. Dispositif selon la revendication 1, **caractérisé en ce que** l'ouverture de sortie (11) est disposée à la base d'un creux.

7. Dispositif selon la revendication 1, **caractérisé en ce que** l'ouverture de sortie (11) est disposée dans une région concave du dispositif.

8. Dispositif selon la revendication 1, **caractérisé en ce qu'**il présente la forme d'un lobe (21) à axe courbe ou coudé, l'ouverture de sortie (11) étant disposée dans une région intérieure de la courbure ou du coude.
